# EUROPÄISCHE PATENTANMELDUNG

(11) **EP 0 750 029 A2**
(43) Veröffentlichungstag der Anmeldung: **27.12.1996**
(21) Anmeldenummer: 96108871.3
(22) Anmeldetag: 03.06.1996
(51) Int. Cl.: C09K 19/34, C09K 19/32, C09K 19/30, C09K 19/20

(54) **Polymerisierbare chirale Verbindungen und deren Verwendung**

(30) Priorität: 09.06.1995 DE 19520704
(71) Anmelder: BASF Aktiengesellschaft, 67063 Ludwigshafen (DE)
(72) Erfinder: Meyer, Frank, Dr., 67063 Ludwigshafen (DE); Siemensmeyer, Karl, Dr., 67227 Frankenthal (DE); Etzbach, Karl-Heinz, Dr., 67227 Frankenthal (DE); Schuhmacher, Peter, Dr., 68163 Mannheim (DE)

(57) **Zusammenfassung**

Polymerisierbare chirale Verbindungen der allgemeinen Formel I

(Z-Y¹-A-O-CO-O-M-Y²)ₙ X I

in der die Variablen folgende Bedeutung haben
- A: Spacer,
- M: mesogene Gruppen
- Y¹,Y²: chemische Bindungen oder die Gruppierungen -O-; -S-; -CO-O-; -O-CO-; -O-CO-O-; -CO-N(R)- oder -N(R)-CO-
- X: ein n-wertiger chiraler Rest
- n: 2 bis 6
- Z: a₁) mindestens einer dieser Reste eine reaktive Gruppe, über die eine Polyaddition herbeigeführt werden kann,
a₂) mindestens zwei dieser Reste Substituenten, die eine reaktive Gruppe tragen, über welche eine Polykondensation herbeigeführt werden kann,
b) Wasserstoff oder nicht-reaktive Reste, soweit die Bedingung (a₁) bzw. (a₂) erfüllt ist,
wobei die Reste Z, Y¹, A, M und Y², da sie n-mal in I enthalten sind, gleich oder verschieden sein können.

Die erfindungsgemäßen Verbindungen eignen sich zur Verwendung in elektro-optischen Anzeigen oder als chirale Dotierstoffe für nematische oder cholesterische Flüssigkristalle zur Erzeugung farbig reflektierender Schichten.

## Beschreibung

Gegenstand der Erfindung sind polymerisierbare chirale Verbindungen der allgemeinen Formel I

(Z-Y¹-A-O-CO-O-M-Y²)ₙ X I

in der die Variablen folgende Bedeutung haben:
- A: Spacer,
- M: mesogene Gruppen
- Y¹,Y²: chemische Bindungen oder die Gruppierungen -O-; -S-; -CO-O-; -O-CO-; -O-CO-O-; -CO-N(R)- oder -N(R) -CO-
- X: ein n-wertiger chiraler Rest
- n: 2 bis 6
- Z: a₁) mindestens einer dieser Reste eine reaktive Gruppe, über die eine Polyaddition herbeigeführt werden kann,
a₂) mindestens zwei dieser Reste Substituenten, die eine reaktive Gruppe tragen, über welche eine Polykondensation herbeigeführt werden kann,
b) Wasserstoff oder nicht-reaktive Reste, soweit die Bedingung (a₁) bzw. (a₂) erfüllt ist,
wobei die Reste Z, Y¹, A, M und Y², da sie n-mal in I enthalten sind, gleich oder verschieden sein können.

Außerdem ist Gegenstand der Erfindung die Verwendung dieser Verbindungen in elektrooptischen Anzeigen oder als chirale Dotierstoffe für nematische oder cholesterische Flüssigkristalle, besonders zur Erzeugung farbig reflektierender Schichten.

Wie für formanisotrope Moleküle bekannt, können beim Erwärmen flüssigkristalline Phasen, sogenannte Mesophasen, auftreten. Die einzelnen Phasen unterscheiden sich durch die räumliche Anordnung der Molekülschwerpunkte einerseits sowie durch die Molekülanordnung hinsichtlich der Längsachsen andererseits (G.W. Gray, P. A. Winsor, Liquid Crystals and Plastic Crystals, Ellis Horwood Limited, Chichester 1974). Die nematisch-flüssigkristalline Phase zeichnet sich dadurch aus, daß lediglich eine Orientierungsfernordnung durch Parallellagerung der Moleküllängsachsen existiert. Unter der Voraussetzung, daß die die nematische Phase aufbauenden Moleküle chiral sind, entsteht eine sogenannte cholesterische Phase, bei der die Längsachsen der Moleküle eine zu ihnen senkrechte, helixartige Überstruktur ausbilden (H. Baessler, Festkörperprobleme XI, 1971). Der chirale Molekülteil kann im flüssigkristallinen Molekül selbst enthalten sein oder aber als Dotierstoff zur nematischen Phase gegeben werden. Durch Dotierung erzeugte Phasen werden als induziert cholesterische Phasen bezeichnet. Dieses Phänomen wurde zuerst an Cholesterinderivaten untersucht (s. z.B. H. Baessler, M.M. Labes, J. Chem. Phys. 52 (1970) 631). Später wurde die Induzierung cholesterischer Phasen auch durch Zusatz anderer chiraler Substanzen möglich, die selbst nicht flüssigkristallin sind (H. Stegemeyer, K.J. Mainusch, Naturwiss. 58 (1971) 599; H. Finkelmann, H. Stegemeyer, Ber. Bunsenges. Phys. Chem. 78 (1974) 869).

Die cholesterische Phase hat bemerkenswerte optische Eigenschaften: eine hohe optische Rotation sowie einen ausgeprägten Circulardichroismus, der durch Selektivreflexion von circularpolarisiertem Licht innerhalb der cholesterischen Schicht entsteht. Die je nach Blickwinkel zu beobachtenden unterschiedlichen Farben sind abhängig von der Ganghöhe der helicalen Überstruktur, die ihrerseits vom Verdrillungsvermögen der chiralen Komponente abhängt. Dabei kann insbesondere durch Änderung der Konzentration eines chiralen Dotierstoffs die Ganghöhe und damit der Wellenlängenbereich des selektiv-reflektierten Lichts einer cholesterischen Schicht variiert werden (J.E. Adams, W.E.L. Haas, Mol. Cryst. Liq. Cryst. 16 (1972) 33). Solche cholesterischen Systeme bieten für eine praktische Anwendung interessante Möglichkeiten. So kann durch Einbau chiraler Molekülteile in mesogene Acrylsäureester nach Orientierung in der cholesterischen Phase und Photovernetzung ein stabiles, farbiges Netzwerk hergestellt werden, dessen Konzentration an chiraler Komponente aber nicht verändert werden kann (G. Galli, M. Laus, A. Angeloni, Makromol. Chem. 187 (1986) 289). Ferner kann durch Zumischen von nichtvernetzbaren chiralen Verbindungen zu nematischen Acrylsäureestern nach Photovernetzung ein farbiges Polymer hergestellt werden (I. Heynderickx, D.J. Broer, Mol. Cryst. Liq. Cryst. 203 (1991) 113), das jedoch noch flüchtige Bestandteile enthält, die für eine technische Anwendung prohibitiv sind.

In der älteren deutschen Patentanmeldung P 43 42 280.2 werden ähnliche polymerisierbare chirale Verbindungen beschrieben, die sich zur Herstellung cholesterischer flüssigkristalliner Polymerisate eignen und die sich von den erfindungsgemäßen Verbindungen im wesentlichen dadurch entscheiden, daß der Spacer A und die mesogene Gruppe M durch andere Gruppen als die Carbonatgruppe verknüpft sind.

Aufgabe der vorliegenden Erfindung war die Synthese neuer chiraler Verbindungen, die zum einen ein hohes Verdrillungsvermögen aufweisen und zum anderen über einen weiten Konzentrationsbereich stabil in die cholesterische Phase eingebaut werden können, ohne daß sie aus der Phase herausdiffundieren oder kristallisieren können.

Demgemäß wurden die eingangs definierten polymerisierbaren chiralen Verbindungen gefunden.

Die in den erfindungsgemäßen Verbindungen enthaltenen Molekülteile Z und A sowie M und X sind über Brückenglieder Y¹ bzw. Y² wie -O-, -S-, -CO-O-, -O-CO-, -O-CO-O-, -CO-N(R) -oder -N(R)-CO- oder auch über eine direkte Bindung miteinander verknüpft, wobei die Verknüpfung des Spacers A mit der mesogenen Gruppe durch eine Carbonatgruppe (-OCOO-) erfolgt. Chirale polymerisierbare Verbindungen mit einer solchen Carbonatgruppe haben die vorteilhafte Eigenschaft, besonders niedrige Phasenumwandlungstemperaturen aufzuweisen und sind somit für Anwendungen bei Raumtemperatur besonders geeignet.

Als Spacer A kommen alle für diesen Zweck bekannten Gruppen in Betracht. Die Spacer enthalten in der Regel 2 bis 30, vorzugsweise 2 bis 12 C-Atome und bestehen aus linearen aliphatischen Gruppen. Sie können in der Kette z.B. durch O, S, NH oder NCH₃ unterbrochen sein, wobei diese Gruppen nicht benachbart sein dürfen. Als Substituenten für die Spacerkette kommen dabei noch Fluor, Chlor, Brom, Cyan, Methyl oder Ethyl in Betracht.

Repräsentative Spacer sind beispielsweise:
-(CH₂)ₚ-, -(CH₂CH₂O)ₘCH₂CH₂-, -CH₂CH₂SCH₂CH₂-, -CH₂CH₂NHCH₂CH₂-, 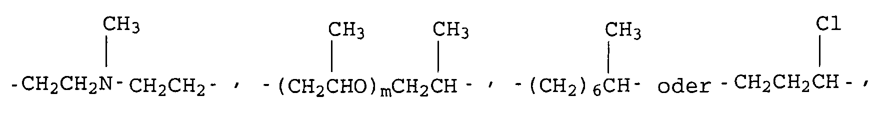 wobei
m 1 bis 3 und
p 1 bis 12 sind.

Als Reste M können alle bekannten mesogenen Gruppen dienen. Insbesondere kommen Gruppen der Formel Ia

-(-T-Y³)ᵣ-T- Ia,

in Betracht, in der die Variablen folgende Bedeutung haben:
- T: zweiwertige isocycloaliphatische, heterocycloaliphatische, isoaromatische oder heteroaromatische Reste,
- Y³: Brückenglieder gemäß der Definition für Y¹; -CH₂-O-; -O-CH₂-; -CH=N- oder -N=CH-
- r: 0 bis 3
wobei die Reste T und Y³ im Falle r > O bzw. r > 1 gleich oder verschieden sein können.

Vorzugsweise ist r 0 oder 1.

Die Reste T können auch durch Fluor, Chlor, Brom, Cyan, Hydroxy oder Nitro substituierte Ringsysteme sein. Bevorzugte Reste T sind:

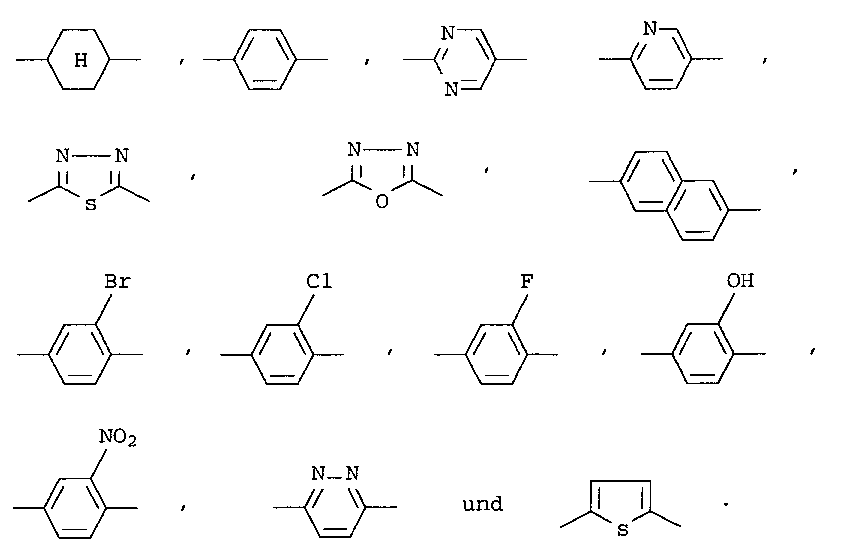

Besonders bevorzugt als mesogene Gruppen M sind z.B.:

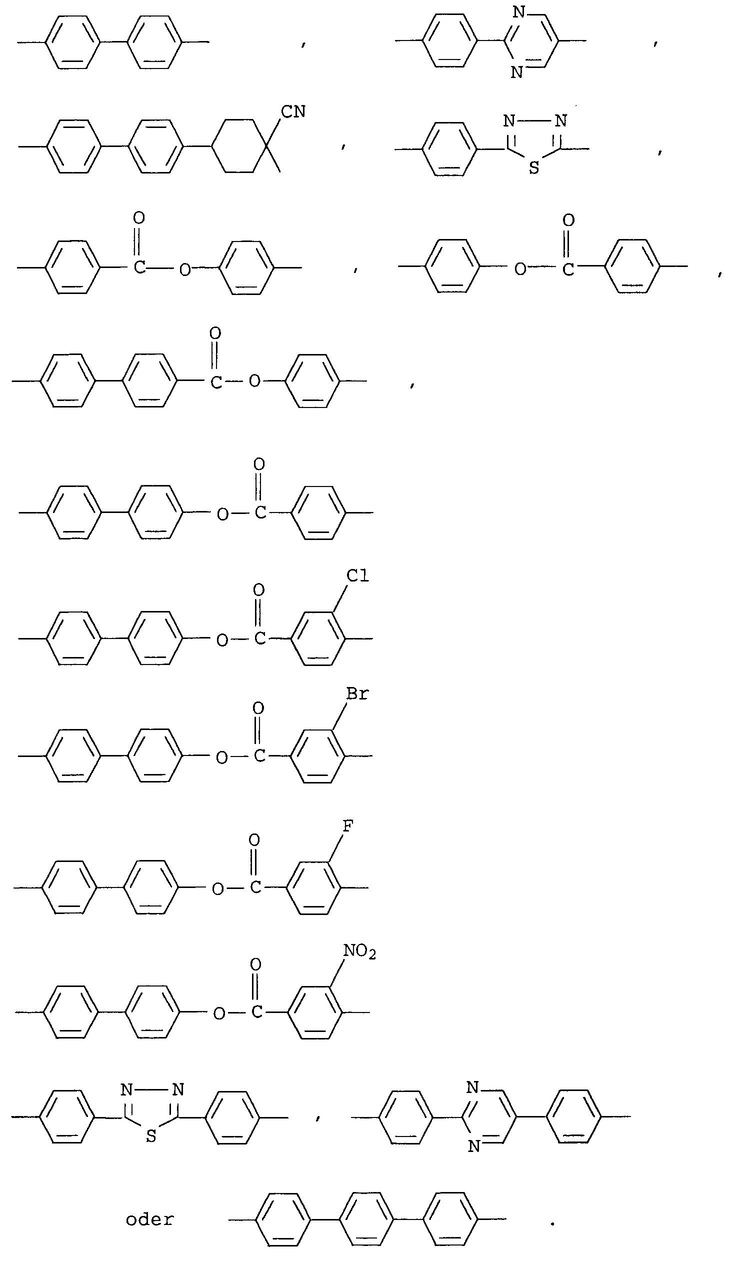

n in Formel I ist vorzugsweise 2 oder 3 und insbesondere 2.

Von den chiralen Resten X sind aufgrund der Verfügbarkeit insbesondere solche bevorzugt, die sich von Zuckern, Binaphthyl- oder Biphenylderivaten sowie optisch aktiven Glykolen, Dialkoholen oder Aminosäuren ableiten. Bei den Zuckern sind insbesondere Pentosen und Hexosen und davon abgeleitete Derivate zu nennen.

Einzelne Reste X sind z.B.: 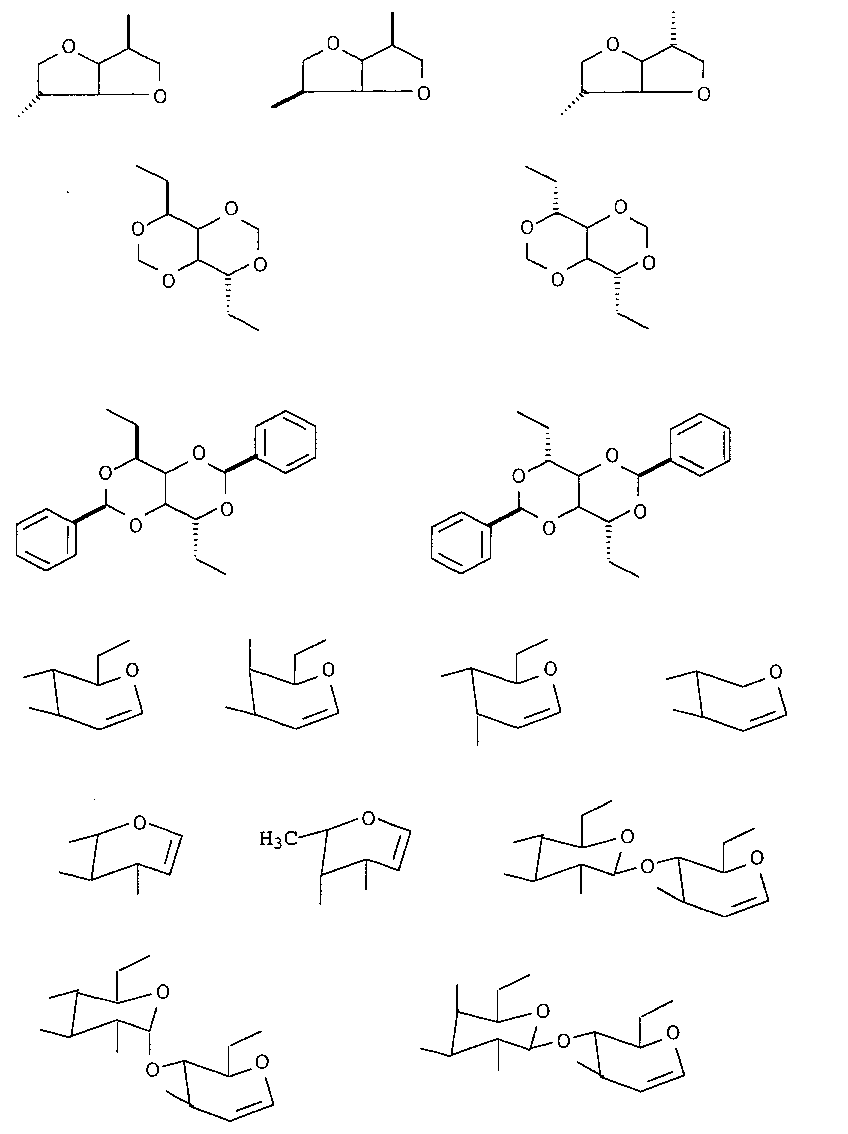 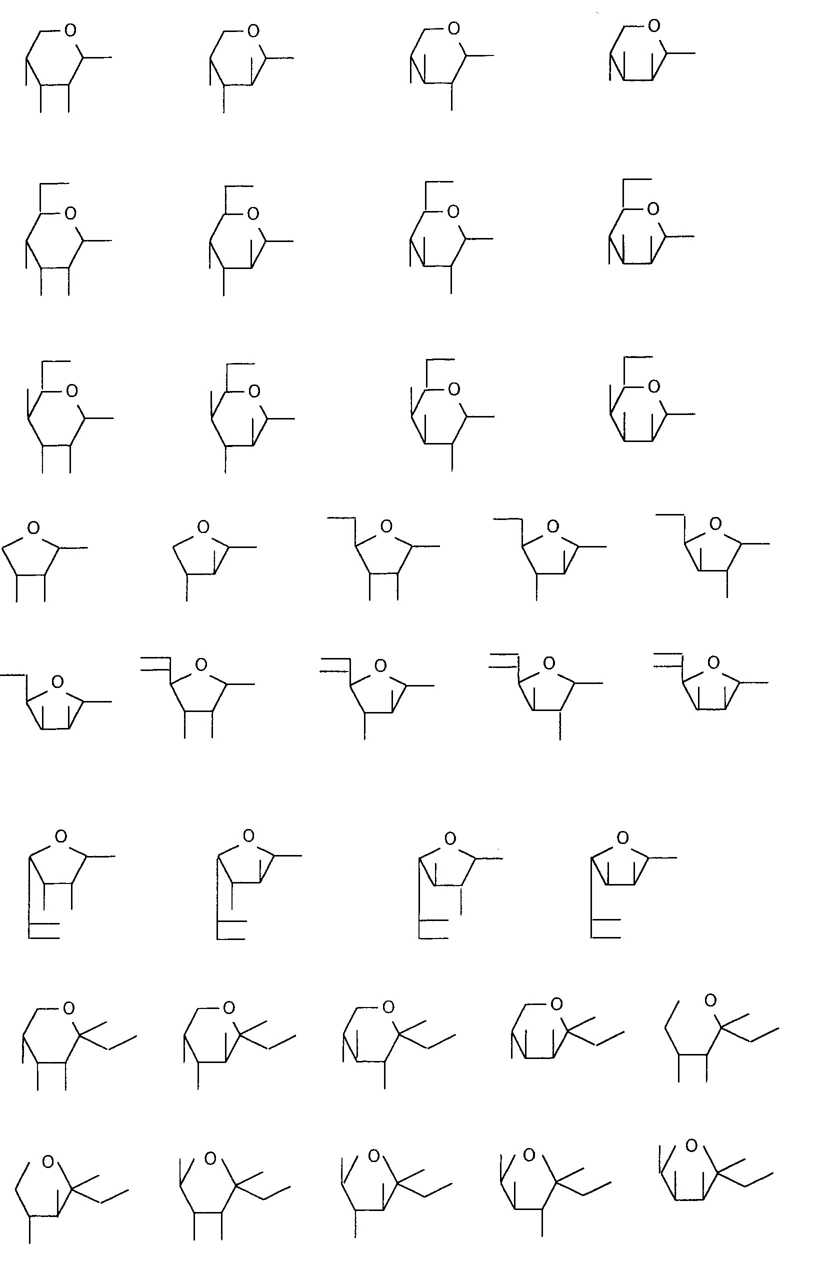 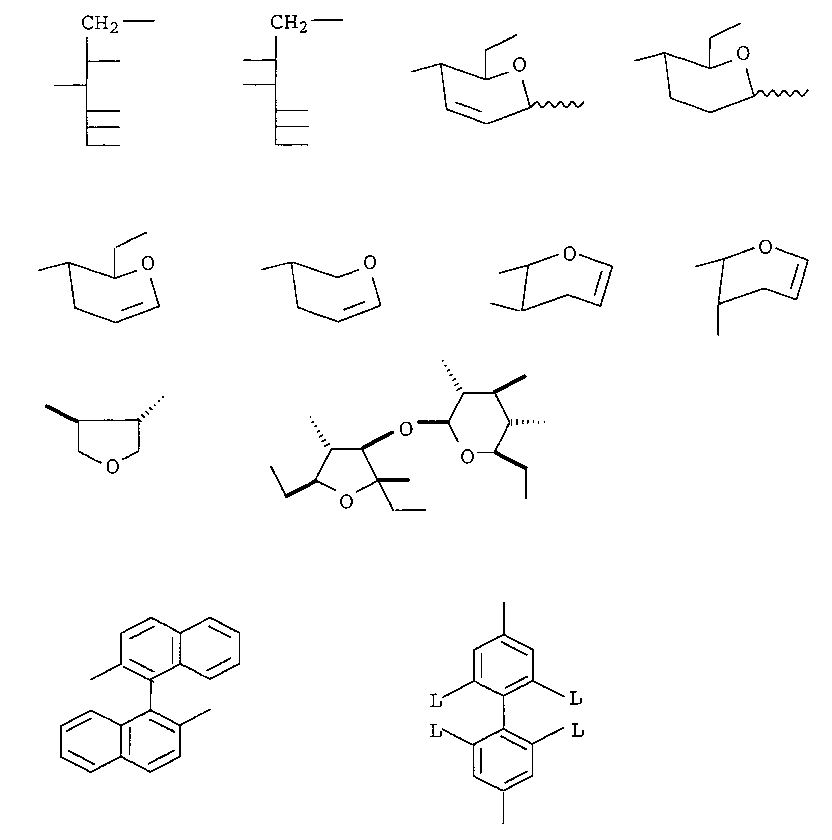 wobei
- L: C₁- bis C₄-Alkyl, C₁-C₄-Alkoxy, Halogen, COOR, OCOR, CONHR oder NHCOR ist und R die angegebene Bedeutung hat.
(Die endständigen Striche in den aufgeführten Formeln geben die freien Valenzen an).

Besonders bevorzugt sind z.B.

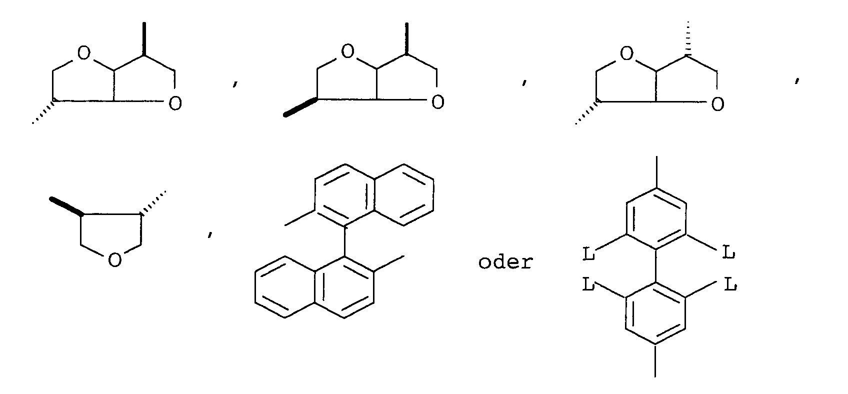

Optisch aktive Glykole oder Derivate davon entsprechen z.B. der Formel 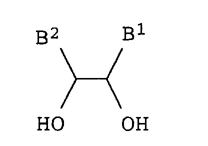 in der die Reste
- B¹ und B²: unabhängig voneinander C₁- bis C₄-Alkyl, das noch durch Hydroxy substituiert und durch -O- unterbrochen sein kann, Phenyl oder gegebenenfalls substituiertes Carboxyl und einer der Reste auch Wasserstoff sind, wobei bei gleichen Resten B¹ und B² die Konfiguration R,S ausgeschlossen ist.

Einzelne derartige Reste B¹ und B² sind z.B.
-CO₂CH₃, -CO₂CH₂CH₃, -CO₂(CH₂)₂CH₃, -CO₂(CH₂)₃CH₃, -CO₂CH(CH₃)₂, -CO₂C(CH₃)₃ oder -CH(OH)CH₂(OH).

Weiterhin sind auch zweiwertige chirale Gruppen als Reste X geeignet, die folgende Strukturen aufweisen:

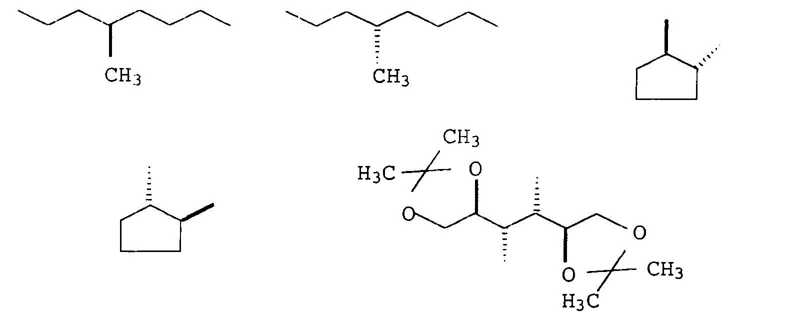

Bevorzugte Reste Z sind:
H₂=CH- , H₂≡C- , 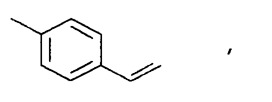 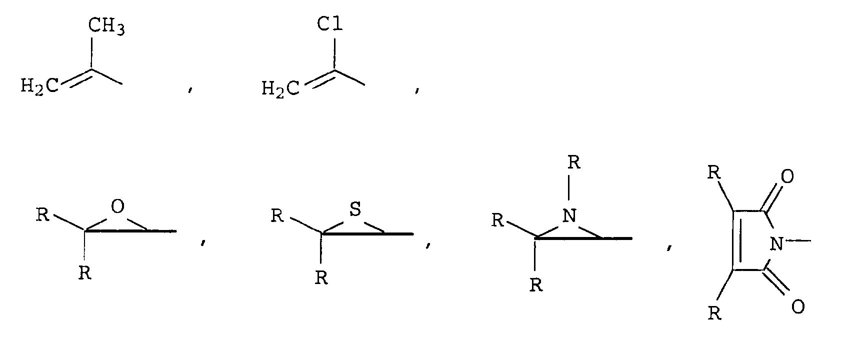 ―N=C=O, ―N=C=S, ―O―C≡N,
―COOH, ―OH oder ―NH₂,
wobei die Reste R gleich oder verschieden sein können. Von den reaktiven polymerisierbaren Gruppen können die Cyanate spontan zu Cyanuraten trimerisieren und sind daher bevorzugt zu nennen. Die anderen genannten Gruppen benötigen zur Polymerisation weitere Verbindungen mit komplementären reaktiven Gruppen. So können beispielsweise Isocyanate mit Alkoholen zu Urethanen und mit Aminen zu Harnstoffderivaten polymerisieren. Analoges gilt für Thiirane und Aziridine. Carboxylgruppen können zu Polyestern und Polyamiden kondensiert werden. Die Maleinimidogruppe eignet sich besonders zur radikalischen Copolymerisation mit olefinischen Verbindungen wie Styrol. Die komplementären reaktiven Gruppen können dabei entweder in einer zweiten erfindungsgemäßen Verbindung vorhanden sein, die mit der ersteren gemischt wird, oder sie können durch Hilfsverbindungen, die 2 oder mehr dieser komplementären Gruppen enthalten, in das Polymerisationsgemisch eingebracht werden.

Da erfindungsgemäße Verbindungen I, in denen mindestens zwei Reste Z reaktive Gruppen bedeuten, die eine Polykondensation- oder Polyaddition eingehen können, besonders stabile Polymerfilme ergeben, sind diese besonders bevorzugt.

Als nichtreaktive Reste Z, welche Bestandteile der erfindungsgemäßen Verbindungen I sein können, soweit mindestens ein Rest Z einen reaktiven Rest bedeutet, eignen sich neben Wasserstoff C₁-C₂₀-Alkylreste, besonders lineare C₁-C₁₂-Alkylreste.

Die chrialen Ausgangsverbindungen für den Molekülteil X, in der Regel Hydroxyverbindungen X(OH)ₙ, sind größtenteils handelsüblich.

Die erfindungsgemäßen Einheiten Z-Y¹-A-O-CO-O-M-Y², sind durch allgemein bekannte Synthesen, wie sie beispielsweise in der DE-A 39 17 196 beschrieben sind, zugänglich.

Die Gruppen Z, A, M und X werden vorzugsweise durch Kondensationsreaktionen aneinander gekoppelt, wobei die gewünschten Brückenglieder Y¹ bzw. Y² ausgebildet werden, also beispielsweise durch Reaktion eines Mesogen-Carboxylates mit einer chiralen Hydroxyverbindung X eine Esterbindung oder durch Kondensation zweier Hydroxygruppen, nach entsprechender Aktivierung, eine Etherbindung u.s.w. Die Carbonatgruppe wird vorzugsweise durch sukzessive Reaktion von Phosgen mit einer hydroxysubstituierten Verbindung Z-Y¹-A-OH und einer Verbindung HO-M oder (HO-M-Y²)ₙX ausgebildet.

Die erfindungsgemäßen Verbindungen eignen sich insbesondere zur Verwendung in elektro-optischen Anzeigeelementen oder als chirale Dotierstoffe für nematische oder cholesterische Flüssigkristalle, insbesondere zur Erzeugung farbig reflektierender Schichten.

### Beispiel 1

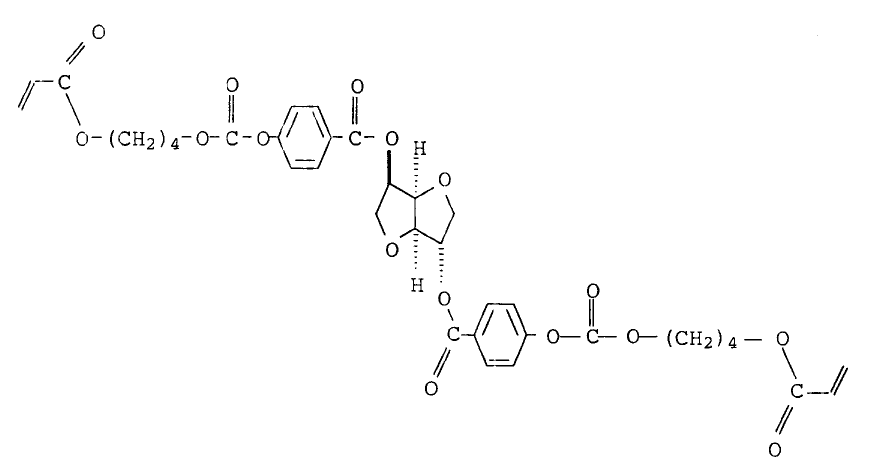

3,5 g (9,1 mmol) Bis-(4'-Hydroxybenzoyl)-isosorbit und 4,15 g (20,1 mmol) 4-Acryloxybutyloxycarbonylchlorid (hergestellt in bekannter Weise aus 4-Acryloxybutanol und Phosgen) in 100 ml Pyridin/Dichlormethan (1:1, Vol:Vol) wurden bei 3°C zur Reaktion gebracht. Es wurde 15 Stunden bei Raumtemperatur nachgerührt, zweimal mit verdünnter Salzsäure und dreimal mit Wasser gewaschen und über Na₂SO₄ getrocknet. Nach Destillation des Lösungsmittel wurde das Produkt chromatographisch gereinigt.
Ausbeute: 24 %.
Schmelzpunkt: 43°C
Helical Twisting Power (HTP) : 34 µm⁻¹
(Die HTP wurde nach der von H. Finkelmann und H. Stegemeyer in Ber. Bunsenges. Phys. Chem. 78, 869 (1974) angegebenen Methode bestimmt)
Analog zu Beispiel 1 wurde die Beispielverbindung 2 hergestellt:

### Beispiel 2

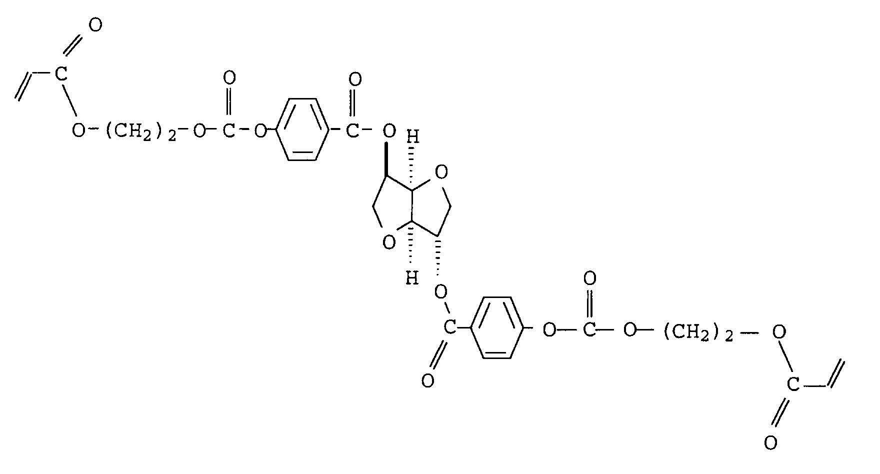

## Patentansprüche

1. Polymerisierbare chirale Verbindungen der allgemeinen Formel
(Z-Y¹-A-O-CO-O-M-Y²)ₙ X I
in der die Variablen folgende Bedeutung haben:
A Spacer,
M mesogene Gruppen
Y¹,Y² chemische Bindungen oder die Gruppierungen -O-; -S-; -CO-O-; -O-CO-; -O-CO-O-; -CO-N(R)- oder -N(R)-CO-
X ein n-wertiger chiraler Rest
R Wasserstoff oder C₁-C₄-Alkylgruppen
n 2 bis 6
Z
a₁) mindestens einer dieser Reste eine reaktive Gruppe, über die eine Polyaddition herbeigeführt werden kann,
a₂) mindestens zwei dieser Reste Substituenten, die eine reaktive Gruppe tragen, über welche eine Polykondensation herbeigeführt werden kann,
b) Wasserstoff oder nicht-reaktive Reste, soweit die Bedingung (a₁) bzw. (a₂) erfüllt ist,
wobei die Reste Z, Y¹, A, M und Y², da sie n-mal in I enthalten sind, gleich oder verschieden sein können.

2. Verbindungen I nach Anspruch 1, in denen n den Wert 2 hat.

3. Verbindungen I nach Anspruch 1 oder 2, in denen die mesogene Gruppe M für eine Gruppe der Formel Ia
-(-T-Y³-)ᵣ-T- Ia
steht, in der die Variablen folgende Bedeutung haben:
T zweiwertige isocycloaliphatische, heterocycloaliphatische, isoaromatische oder heteroaromatische Reste
Y³ Brückenglieder gemäß der Definition für Y¹; -CH₂-O-; -O-CH₂-; -CH=N- oder -N=CH-
r 0 bis 3,
wobei die Reste T und Y³ im Falle r > 0 bzw. r > 1 gleich oder verschieden sein können.

4. Verbindungen I nach Anspruch 3, in denen r den Wert 0 oder 1 hat.

5. Verbindungen I nach den Ansprüchen 2 bis 4, in denen X eine der folgenden Gruppen bedeutet: 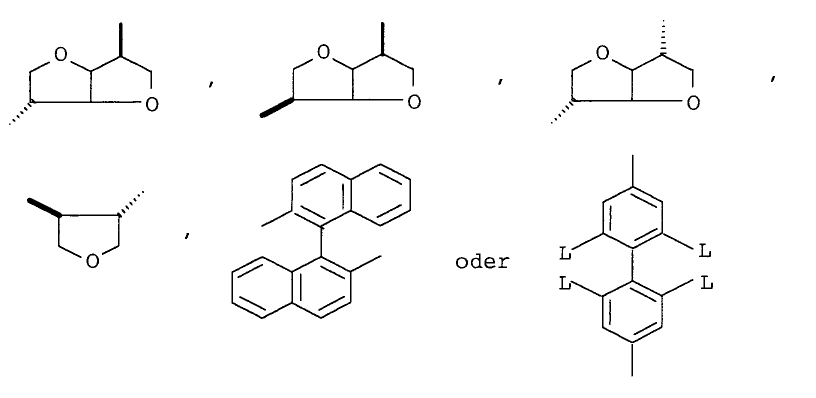 wobei
L für gleiche oder verschiedene der folgenden Substituenten steht: C₁-C₄-Alkyl oder -Alkoxy, Halogen, -CO-OR, -O-CO-R, -CO-NH-R oder -NH-CO-R.

6. Verbindungen I nach den Ansprüchen 1 bis 5, in denen mindestens eine der Gruppierungen Z-Y¹- für die Cyanat- oder Isocyanatgruppe stehen, wobei Y¹ in diesen Fällen eine chemische Bindung bedeutet.

7. Verbindungen I nach den Ansprüchen 1 bis 5, in denen mindestens einer der Reste Z eine Epoxygruppe bedeutet oder ein Rest ist, der eine Epoxygruppe trägt.

8. Verbindungen I nach den Ansprüchen 1 bis 7, in denen die Reste Z, Y¹, A, M, Y², R und L jeweils gleich sind.

9. Verwendung der Verbindungen I gemäß den Ansprüchen 1 bis 8 in elektrooptischen Flüssigkristall-Anzeigevorrichtungen.

10. Verwendung der Verbindungen I gemäß den Ansprüchen 1 bis 8 als chirale Dotierstoffe für nematische oder cholesterische Flüssigkristalle.

11. Verwendung der Verbindungen I gemäß den Ansprüchen 1 bis 8 als chirale Dotierstoffe zur Erzeugung farbig reflektierender cholesterisch flüssigkristalliner Schichten.
